Europäisches Patentamt

European Patent Office

Office européen des brevets

(11) Publication number: **0 106 927**
**B1**

(12) EUROPEAN PATENT SPECIFICATION

(45) Date of publication of patent specification: **22.07.87**

(21) Application number: **82305760.9**

(22) Date of filing: **29.10.82**

(51) Int. Cl.⁴: **B 01 J 27/18,** C 07 C 51/25,
C 07 C 51/215

(54) Production of improved mixed vanadium phosphorus oxide catalysts.

(43) Date of publication of application:
**02.05.84 Bulletin 84/18**

(45) Publication of the grant of the patent:
**22.07.87 Bulletin 87/30**

(84) Designated Contracting States:
**DE FR GB IT NL**

(56) References cited:
**EP-A-0 039 537**
**EP-A-0 056 183**
**FR-A-2 276 874**
**US-A-4 016 105**
**US-A-4 017 521**
**US-A-4 043 943**
**US-A-4 132 670**
**US-A-4 187 235**
**US-A-4 244 879**
**US-A-4 315 864**
**US-A-4 328 126**

(73) Proprietor: **THE STANDARD OIL COMPANY**
**Midland Building**
**Cleveland, Ohio 44115 (US)**

(72) Inventor: **Bremer, Noel Jerome**
**3633 By Lane**
**Kent Ohio 44240 (US)**
Inventor: **Weber, Andrew M.**
**24600 Scarlet Oak Drive**
**Bedford Heights Ohio 44146 (US)**
Inventor: **Dria, Dennis Edward**
**3604 Ambleside Drive**
**Austin Texas 78759 (US)**

(74) Representative: **Smith, Sydney et al**
**Elkington and Fife High Holborn House 52/54**
**High Holborn**
**London WC1V 6SH (GB)**

Courier Press, Leamington Spa, England.

## 0 106 927

**Description**

This invention relates to the production of improved mixed vanadium phosphorus oxide catalysts and to the use thereof in oxidation processes, for example in the production of dicarboxylic acid anhydrides by the oxidation of hydrocarbons; more particularly, it relates to the production of such catalysts which are suitable for producing maleic anhydride from 4-carbon hydrocarbons, such as $n$-butane, $n$-butenes, 1,3-butadiene or mixtures thereof.

Catalysts containing vanadium and phosphorus oxides have been used in the oxidation of 4-carbon hydrocarbons, such as $n$-butane, $n$-butenes, 1,3-butadiene or mixtures thereof, using molecular oxygen or free oxygen-containing gases to produce maleic anhydride. Conventional processes for the production of these catalysts involve reducing a pentavalent vanadium compound and combining the same with a phosphorus compound and, if desired, promoter element compounds under conditions which will provide or maintain vanadium in a valence state below +5 to form catalyst precursors capable of being converted to an oxide. The catalyst oxide precursor is then recovered and calcined to provide active catalytic material.

The use of gaseous HCl as a reducing agent for vanadium is disclosed in U.S. Patent No. 4,002,650 where the vanadium and phosphorus components are reacted in an aqueous solution. The use of gaseous HCl as a reducing agent for vanadium is also described in U.S. Patent No. 4,043,943 where the vanadium and phosphorus components are reacted in liquid organic medium.

In US—A—4,017,521 there is also described a process for the preparation of a vanadium-phosphorus mixed oxide in which vanadium pentoxide is added to an organic medium, for example isobutanol, and anhydrous hydrogen chloride passed into the slurry to dissolve the vanadium compound. The resulting red-brown solution which is nearly saturated with hydrogen chloride gas is added to a solution of orthophosphoric acid. After the introduction of the red-brown solution to the phosphoric acid solution the temperature of the resulting solution is increased to reflux temperature i.e. about 110°C and is maintained for about 1.5 hours. Thereafter, the reflux condenser is removed and isobutanol solvent is distilled from the reaction mixture. The catalyst precursor precipitates, is dried and then activated by heating.

U.S. Patent No. 4,016,105 describes the production of vanadium and phosphorus oxide-containing catalysts utilizing as reducing agents organic acids or aldehydes, together with a co-reducing secondary alcohol. These reducing agents are added to an aqueous solution with the vanadium and phosphorus components.

Similar techniques are described in European Patent Application EP—A—3,431 in which the additional step of comminuting the vanadium-phosphorus precursor to a particle size of from 0.5 to 0.7 mm (from 500 to 700 microns) is disclosed.

The use of such reducing agents as disclosed in the art, requires particular precautions, in the production of these catalysts because of the corrosive nature of the materials utilized. The process according to the present invention permits the production of mixed vanadium phosphorus oxide catalysts without the use of corrosive reducing agents.

A process for the production of catalysts containing vanadium and phosphorus oxides was described in U.S. Patent No. 4,132,670 which required the maintenance of a solid phase and dispersion of the vanadium-containing compound dispersion in an organic liquid medium, such as alcohols, aldehydes, ketones, ethers or mixtures thereof, heating the dispersion to reduce the vanadium and thereafter adding phosphoric acid in an organic solvent.

The production of oxidation catalysts containing the mixed oxides of vanadium and phosphorus is disclosed in U.S. Patent No. 4,244,879 wherein a vanadium compound is at least partially solubilized in an organic liquid medium capable of reducing at least a portion of the vanadium to a +4 valence state and unsolubilized vanadium having a particle size larger than 0.1 mm diameter is removed from the medium before addition of a phosphorus-containing compound.

The preparation of vanadium phosphorus mixed oxide containing catalysts is disclosed in EP—A—39537 wherein partial reduction of a pentavalent vanadium compound is effected in the presence of a phosphorus compound in an organic liquid medium capable of reducing the vanadium. EP—A—56183 discloses the production of vanadium phosphorus oxide catalysts utilizing a mixed phosphorus component compound source, preparing the catalyst in a liquid medium capable of reducing the vanadium component.

In the production of vanadium phosphorus mixed oxide catalysts utilizing organic liquid reaction media, it had previously been known to remove excess water formed as a result of the reaction of vanadium by utilizing a Dean-Stark receiver. Such devices at least partially remove the aqueous phase from the reaction medium, by evaporation, returning condensed organic phase to the reaction media.

It has now been found, however, that vanadium phosphorus mixed oxide catalysts having enhanced activity for oxidation reactions, such as the production of maleic anhydride from 4-carbon hydrocarbons, such as $n$-butane, are obtained in organic liquids, selected from alcohols and glycols, capable of reducing the valence state of at least a portion of pentavalent vanadium to +4, by reacting the vanadium component and phosphorus component in the organic liquid containing reaction medium, preferably with heating, and removing at least 1.5 moles of the organic liquid per mole of vanadium reacted.

It is therefore an object of the present invention to provide a process for the production of vanadium and phosphorus-containing catalysts which are particularly useful for the oxidation of 4-carbon

2

**0 106 927**

hydrocarbons to produce maleic anhydride, which catalysts exhibit excellent yields and selectivity to maleic anhydride.

It is a further object of the present invention to provide a process for the production of vanadium and phosphorus-containing catalysts which are particularly useful for the oxidation of 4-carbon hydrocarbons to produce maleic anhydride which is simplified, highly reproducible and economical, which avoids the hazards of corrosion and which is capable of commercial scale-up.

The present invention provides a process for the production of a vanadium phosphorus mixed oxide containing catalyst comprising the following steps:

(a) introducing a pentavalent vanadium compound into an organic liquid reaction medium selected from alcohols and glycols capable of reducing at least a portion of the vanadium to a valance state of +4;

(b) introducing at least one pentavalent phosphorus compound into the reaction medium;

(c) effecting reduction of the vanadium by heating under reflux prior or subsequent to the addition of the phosphorus compound and reacting the vanadium with the phosphorus compound to form a catalyst precursor;

(d) recovering the catalyst precursor from the reaction medium;

(e) drying the catalyst precursor; and

(f) calcining the catalyst precursor; characterised in that (1) no corrosive reducing agent is added to the organic liquid reaction medium so that it is not a solvent for the catalyst precursor, and (2) during the reduction of the vanadium by heating under reflux according to step (c), at least 1.5 moles of organic liquid medium is removed per mole of vanadium reduced or reacted.

The catalysts produced by the above process are particularly effective in the oxidation of 4-carbon hydrocarbons, such as $n$-butane, $n$-butenes, 1,3-butadiene or mixtures thereof, using molecular oxygen or a free oxygen-containing gas in the vapour phase to produce high yields of maleic anhydride with high selectivity. Essentially all the product produced in this oxidation process is maleic anhydride, with only minor amounts of lower acids being detected.

In the present process for the production of an oxidation catalyst containing the mixed oxides of vanadium and phosphorus, a vanadium compound, particularly a pentavalent vanadium compound, is introduced into a liquid reaction medium capable of reducing the valence state of the vanadium. Suitable vanadium compounds containing pentavalent vanadium include: vanadium pentoxide or vanadium salts, such as ammonium metavanadate. Vanadium pentoxide is preferred.

According to a preferred embodiment of the present invention, at least one phosphorus-containing compound is added to the reaction medium before substantial reduction of the vanadium to a valence state of less than +5 is effected. The phosphorus compounds utilized in the process according to the present invention are preferably pentavalent and suitable phosphorus compounds containing pentavalent phosphorus include: phosphoric acid, phosphorus pentoxide or a mixed pentavalent phosphorus component comprising a mixture of orthophosphoric acid and pyrophosphoric acid. Optionally, minor amounts of higher polyphosphoric acids may be included. The phosphorus component mixture should comprise from 45 to 90 percent orthophosphoric acid, from 10 to 50 percent pyrophosphoric acid and from 0 to 10 percent triphosphoric acid and higher polyphosphoric acids, percentages being based upon weight of total phosphoric acids. As hydrolysis is a factor in determining the ratio of orthophosphoric acid to pyrophosphoric acid when present in aqueous solution, the above weight ratios are significant provided an extended period of hydrolysis has not occurred to convert the pyrophosphoric acid and higher polyphosphoric acids to the orthophosphoric form.

The phosphorus-containing compound or compounds may be added to the vanadium/liquid reaction medium in the form of a solution of the phosphorus component in either a component of the liquid reaction medium or in a liquid capable of yielding the phosphorus component to the liquid reaction mixture. Alternatively, a vanadium compound and a phosphorus compound, such as 100% phosphoric acid, may be introduced simultaneously into the liquid reaction medium. In another embodiment, the vanadium compound is introduced into a solution or dispersion of the phosphorus component in the liquid reaction medium.

It is preferred that the vanadium-containing compound which is introduced into the liquid medium have a small particle size and means for further reducing particle size of the vanadium compound while in the liquid medium, such as by ball milling the initial suspension of vanadium in the liquid medium, may be employed.

The organic liquid medium employed in the process according to the present invention must be capable of reducing at least a portion of the vanadium to a +4 valence state, preferably upon mixing and heating. In addition, the liquid medium should be a solvent for phosphoric acid and be relatively unreactive towards phosphoric acid. The liquid medium should not, however, be a solvent for the mixed oxide precursor of vanadium and phosphorus and thus the medium is maintained free of corrosive reducing or solubilizing agents, such as HCl, HBr and oxalic acid. Suitable liquid media for use in accordance with the present invention include alcohols and glycols and are preferably anhydrous. Examples of organic liquids suitable for use in the present process are isobutanol and ethylene glycol.

After the pentavalent vanadium compound is introduced into the liquid reaction medium, reduction of the vanadium is effected either prior or subsequent to the addition of the phosphorus component to the liquid reaction medium. The reduction is preferably effected by heating the reaction medium, with stirring

3

if desired. Preferred vanadium and phosphorus oxide catalysts for the oxidation of 4-carbon hydrocarbons to maleic anhydride contain vanadium in an average valence state of from +3.5 to +4.6. This average valence state is achieved when at least a portion of the pentavalent vanadium introduced into the reaction mixture is reduced to the +4 state. The average valence state of the vanadium is reduced preferably to about +4.1. After partial reduction of the vanadium, in one embodiment of the present invention where reduction is effected prior to the addition of the phosphorus component to the reaction medium, large particle unsolubilized vanadium-containing compounds may be removed from the reaction mixture as taught in U.S. Patent No. 4,244,879.

According to the present invention, the reduction of the pentavalent vanadium is carried out in the organic liquid reaction medium with heating under reflux conditions, and preferably in the presence of the phosphorus component. Vanadium phosphorus mixed oxide catalysts having improved activity for the oxidation of 4-carbon hydrocarbons, such as $n$-butane, are obtained when a portion of the organic liquid is removed from the reaction medium during the reduction reaction of vanadium and/or the reaction of vanadium and the phosphorus component. Such removal is effected during heating under reflux. At least 1.5 moles organic liquid per mole of vanadium reacted should be removed during the reaction of vanadium and removal of from 1.5 to 15 moles of organic liquid per mole of vanadium reacted is preferred.

The removal of the organic liquid by distillation under reflux, effects the removal not only of the initial organic liquid utilized, but additionally effects the removal of by-product liquids of the reaction, such as water, aldehydes and other oxidation products of the organic liquid utilized. The number of moles of organic liquid to be removed, therefore, should be deemed to include the number of moles of by-product organic liquids which would also be removed. It is preferred that the removal of the organic liquid and by-products commence as soon as practicable upon the initiation of the reaction of the vanadium.

The vanadium phosphorus catalyst precursor which is formed in the present process is a finely divided precipitate or powder. The catalyst precursor, when dried, forms a freely flowing powdery to granular solid, suitable for economical and commercial processing. After the catalyst precursor is formed, it is recovered from the reaction medium by conventional methods including evaporation, filtration, centrifugation and decantation. The catalyst precursor or catalyst precursor precipitate is dried and thereafter calcined.

It is within the scope of the present invention to include promoter element-containing compounds in the reaction mixture at a suitable point, either prior or subsequent to reduction of the vanadium, in order that the catalyst precursor or catalyst precursor precipitate contain the promoter element. Suitable promoters include U, Co, Mo, Fe, Zn, Hf, Zr or mixtures thereof.

Catalysts obtained according to the present invention exhibit a phosphorus to vanadium ratio of from 0.9:1 to 1.3:1. The catalyst is calcined in an inert atmosphere, air or an oxygen-containing gas at a temperature of from 250 to 600°C, generally for a period of up to 5 hours or more. Calcination of the catalyst may be accomplished by heating the catalyst in a mixture of steam and air or air alone over the catalyst at a temperature of from 300 to 500°C generally for a period of from 1 to 5 hours. The catalyst may also be calcined either in the presence of hydrocarbon, an inert gas, or both.

The hydrocarbon reacted to form maleic anhydride may be $n$-butane, the $n$-butenes, 1,3-butadiene or mixtures thereof. Preferred is the use of $n$-butane or a mixture of hydrocarbons that are produced in refinery streams. The molecular oxygen used for the reaction to produce maleic anhydride is most conveniently added as air, but synthetic streams containing molecular oxygen are also suitable. In addition to the hydrocarbon and molecular oxygen, other gases may be added to the reactant feed. For example, steam or nitrogen may be added to the reactants.

The ratio of the reactants may vary widely and is not critical. The ratio of molecular oxygen to the hydrocarbon may range from 3 to 30 moles of oxygen per mole of hydrocarbon. Preferred oxygen/hydrocarbon ratios are from 4 to 20 moles of oxygen per mole of hydrocarbon.

The reaction temperature may vary widely and is dependent upon the particular hydrocarbon and catalyst employed. Normally, temperatures of from 250 to 600°C are employed, with temperatures of from 325 to 475°C being preferred.

The catalyst may be used alone or a support may be employed. Suitable supports include silica, alumina, "Alundum"®, silicon carbide, titania, boron phosphate and zirconia. The catalyst may be used in a fixed-bed reactor using tablets or pellets, for example, or in a fluid-bed reactor using catalysts prepared, such as by spray drying, and preferably having a particle size of less than 0.3 mm (less than 300 microns). The contact time may be as low as a fraction of a second or as high as 50 seconds. The reaction may be conducted at atmospheric, super-atmospheric or sub-atmospheric pressure.

The present invention is illustrated by the following:

Examples 1 to 3

Catalysts corresponding to the formula $V_1P_{1.2}O_x$ (wherein x represents the number of oxygen atoms required to satisfy the valencies of the other elements) were prepared as follows.

909.5 g $V_2O_5$ and 1384 g $H_3PO_4$ (85%) were introduced into 16 litres isobutanol with stirring. The reaction mixture was heated to reflux and distillation of the organic liquid was commenced and continued until about 9 litres were removed. Reflux was continued for about 9 hours. The resulting catalyst precursor was removed from the reaction mixture by filtration and was dried for about 2 hours at 150°C. The dried precursor was calcined in air for 1 hour at 400°C. The resulting catalyst was processed with 3% stearic acid to form 0.48 cm (3/16th inch) tablets.

Comparative examples 4 and 5

Catalysts corresponding to the formula $V_1P_{1.2}O_x$ were prepared according to the procedure of Examples 1 to 3, except that the reaction mixture was not subjected to distillation.

Example 6

A catalyst corresponding to the formula $V_1P_{1.2}O_x$ was prepared by the procedure of Examples 1 to 3, except that the phosphorus component added was 1307 g of a mixture of orthophosphoric acid (87%, by weight), pyrophosphoric acid (11.5%, by weight) and triphosphoric acid (1.5%, by weight). Distillation of the reaction mixture was begun after about 1 hour of refluxing and was continued until about 2.7 litres of organic liquid was removed.

Examples 7 and 8

Catalysts corresponding to the formula $V_1P_{1.2}O_x$ were prepared according to the procedure of Example 6, except that distillation was commenced after about 1/2 hour of refluxing and was continued until about 6 litres of organic liquid was removed.

Examples 9 and 10

Catalysts corresponding to the formula $V_1P_{1.2}O_x$ were prepared according to the procedure of Example 6, except that about 8 litres of organic liquid were removed by distillation.

Examples 11 and 12

Catalysts corresponding to the formula $V_1P_{1.2}O_x$ were prepared according to the procedure of Example 6, except that about 11 litres of organic liquid were removed by distillation.

Comparative examples 13 to 16.

Catalysts corresponding to the formula $V_1P_{1.2}O_x$ were prepared according to the procedure of Example 6, except that no distillation of organic liquid was effected.

The catalysts described in Examples 1 to 16 were tested for the production of maleic anhydride from $n$-butane using a 20 ml fixed-bed reactor consisting of a 38cm length of stainless steel tubing having an outer diameter of about 1.3 cm and having a full length 0.31 cm axial thermowell. The reactor was heated using a split stainless steel block furnace. Flasks for receiving the product maleic anhydride were mounted in ice water and tail gasses were routed to a Carle Analytical Gas Chromatograph III for analysis. Reaction conditions and results of the tests run are described in the Table below. The results are stated in terms as follows:

$$\text{Single pass yield} = \frac{\text{Moles of maleic anhydride formed}}{\text{Moles of butane fed}} \times 100$$

$$\text{Total conversion} = \frac{\text{Moles of butane reacted}}{\text{Moles of butane fed}} \times 100$$

$$\text{Selectivity} = \frac{\text{Single pass yield} \times 100}{\text{Total conversion}}$$

As may be seen from the results reported in the Table, vanadium phosphorus mixed oxide containing catalysts produced according to the present invention, at least 1.5 moles of organic liquid per mole of vanadium reacted being removed from the reaction medium during the reduction and/or reaction of vanadium, exhibit unexpectedly improved activity for the production of maleic anhydride from 4 carbon hydrocarbons, such as butane, as compared to catalysts produced without such reaction liquid removal.

The liquid removed generally contains a portion of the oxidation products of the organic liquid utilized. As an example, when isobutanol was utilized as the organic liquid reaction medium for a catalyst preparation in which about 3 litres of isobutanol and by-products (32.7 moles) were removed from the reaction medium in which 10 moles of vanadium were reacted for a removal mole ratio of about 3.3, the by-products included about 46 g isobutyraldehyde (0.64 moles), 25 g acetone (0.43 moles) and 223 g water. The by-products are easily separated from the desired organic liquid alcohol or glycol, and the purified organic liquid may be recycled for use in the catalyst preparation.

Both the uncalcined vanadium phosphorus catalyst precursors and the calcined vanadium phosphorus mixed oxide catalysts produced according to the present invention with organic liquid removal exhibit higher intrinsic surface areas as compared to the corresponding precursors and catalysts obtained without

# 0 106 927

such organic liquid removal. For example, the intrinsic surface area of the catalyst precursor of Example 7 was 20% greater than the intrinsic surface area of the catalyst precursor of Comparative Example 14 and the intrinsic surface area of the calcined catalyst of Example 7 was nearly 200% greater than the intrinsic surface area of the calcined catalyst of Comparative Example 14. An increase in the intrinsic surface area of vanadium phosphorus mixed oxide catalyst of this type generally indicates an increase in oxidative catalytic activity. This increased activity is evidenced additionally by the results reported in the Table in which increased conversions and yields of maleic anhydride are exhibited by catalysts obtained according to the present invention, as compared to the Comparative Examples.

## TABLE
### Production of maleic anhydride from $n$-Butane over $V_1P_{1.2}O_x$ catalysts

| Example number | Temperature (°C) | Contact time (seconds) | % conversion | Maleic anhydride | | Removal mole ratio* |
| --- | --- | --- | --- | --- | --- | --- |
| | | | | % Yield | % Selectivity | |
| 1 | 402 | 2 | 80.8 | 52.0 | 64.3 | 10 |
| 2 | 402 | 2 | 76.6 | 51.4 | 67.1 | 10 |
| 3 | 409 | 2 | 81.6 | 53.3 | 65.4 | 10 |
| C4 | 400 | 2 | 66.6 | 45.4 | 68.1 | — |
| C5 | 400 | 2 | 66.3 | 46.4 | 70.1 | — |
| 6 | 423 | 1 | 88.5 | 51.6 | 58.3 | 3 |
| 7 | 418 | 1 | 82.6 | 52.3 | 63.3 | 6.5 |
| 8 | 401 | 2 | 87.0 | 58.6 | 67.3 | 6.5 |
| 9 | 420 | 1 | 78.9 | 47.5 | 60.2 | 8.7 |
| 10 | 409 | 2 | 83.4 | 53.5 | 64.1 | 8.7 |
| 11 | 420 | 1 | 85.7 | 46.7 | 54.5 | 12 |
| 12 | 400 | 2 | 84.6 | 55.8 | 66.1 | 12 |
| C13 | 420 | 1 | 60.0 | 33.5 | 55.9 | — |
| C14 | 419 | 1 | 74.2 | 48.2 | 64.9 | — |
| C15 | 407 | 2 | 64.9 | 41.2 | 63.5 | — |
| C16 | 402 | 2 | 69.8 | 46.9 | 67.2 | — |

*Moles organic liquid removed per mole of vanadium reacted in catalyst production

## Claims

1. A process for the production of a vanadium phosphorus mixed oxide containing catalyst comprising the following steps:

(a) introducing a pentavalent vanadium compound into an organic liquid reaction medium selected from alcohols and glycols capable of reducing at least a portion of the vanadium to a valence state of +4;

(b) introducing at least one pentavalent phosphorus compound into the reaction medium;

(c) effecting reduction of the vanadium by heating under reflux prior or subsequent to the addition of the phosphorus compound and reacting the vanadium with the phosphorus compound to form a catalyst precursor;

(d) recovering the catalyst precursor from the reaction medium;

(e) drying the catalyst precursor; and

(f) calcining the catalyst precursor; characterised in that (1) no corrosive reducing agent is added to the organic liquid reaction medium so that it is not a solvent for the catalyst precursor, and (2) during the reduction of the vanadium by heating under reflux according to step (c), at least 1.5 moles of organic liquid medium is removed per mole of vanadium reduced or reacted.

6

2. A process as claimed in claim 1 characterised in that from 1.5 to 15 moles of organic liquid is removed per mole of vanadium reduced or reacted.

3. A process as claimed in claim 1 or claim 2 characterised in that the catalyst additionally comprises promoter elements selected from U, Co, Mo, Fe, Zn, Hf, Zr and mixtures thereof.

4. A process as claimed in any of claims 1 to 3, characterised in that the phosphorus compound is added to the reaction medium before substantial reduction of vanadium occurs.

5. A process as claimed in any of claims 1 to 4 characterised in that the phosphorus compound comprises orthophosphoric acid.

6. A process as claimed in any of claims 1 to 5 characterised in that the phosphorus compound comprises a mixture of orthophosphoric acid and pyrophosphoric acid.

7. A process as claimed in any of claims 1 to 6 characterised in that the organic liquid comprises isobutanol and/or ethylene glycol.

**Patentansprüche**

1. Verfahren zur Herstellung eines Katalysators, der ein Vanadium-Phosphor-Mischoxid enthält, das die folgenden Schritte umfaßt:

(a) Einführen einer fünfwertigen Vanadiumverbindung in ein flüssiges organisches Reaktionsmedium, ausgewählt aus Alkoholen und Glykolen, das in der Lage ist, wenigstens einen Teil des Vanadiums zu einer Wertigkeitsstufe von +4 zu reduzieren;

(b) Einführen wenigstens einer fünfwertigen Phosphorverbindung in das Reaktionsmedium;

(c) Bewirken der Reduktion des Vanadiums durch Erhitzen unter Rückfluß vor oder nach der Zugabe der Phosphorverbindung und Reaktion des Vanadiums mit der Phosphorverbindung, um einen Katalysatorvorläufer zu bilden;

(d) Abtrennen des Katalysatorvorläufers aus dem Reaktionsmedium;

(e) Trocknen des Katalysatorvorläufers; und

(f) Kalzinieren des Katalysatorvorläufers; dadurch gekennzeichnet, daß (1) kein korrosives Reduktionsmittel zu dem flüssigen organischen Reaktionsmedium zugeführt wird, so daß es kein Lösungsmittel des Katalysatorvorläufers ist, und (2) daß während der Reduktion des Vanadiums durch Erhitzen unter Rückfluß nach Schritt (c) wenigstens 1,5 Mol des flüssigen organischen Mediums auf 1 Mol des reduzierten und abreagierten Vanadiums entfernt wird.

2. Verfahren nach Anspruch 1, dadurch gekennzeichnet, daß 1,5 bis 15 Mol der organischen Flüssigkeit auf 1 Mol des reduzierten oder abreagierten Vanadiums entfernt werden.

3. Verfahren nach Anspruch 1 oder 2, dadurch gekennzeichnet, daß der Katalysator zusätzlich Katalysebeschleunigerelemente umfaßt, die von U, Co, Mo, Fe, Zn, Hf, Zr und Mischungen davon ausgewählt werden.

4. Verfahren nach einem der mehreren der Ansprüche 1 bis 3, dadurch gekennzeichnet, daß die Phosphorverbindung zu dem Reaktionsmedium zugefügt wird, bevor eine wesentliche Reduktion des Vanadiums stattfindet.

5. Verfahren nach einem oder mehreren der Ansprüche 1 bis 4, dadurch gekennzeichnet, daß die Phosphorverbindung Orthophosphorsäure umfaßt.

6. Verfahren nach einem oder mehreren der Ansprüche 1 bis 5, dadurch gekennzeichnet, daß die Phosphorverbindung eine Mischung von Orthophosphorsäure und Pyrophosphorsäure umfaßt.

7. Verfahren nach einem oder mehreren der Ansprüche 1 bis 6, dadurch gekennzeichnet, daß die organische Flüssigkeit Isobutanol und/oder Ethylenglykol umfaßt.

**Revendications**

1. Procédé de production d'un catalyseur contenant un oxyde mixte de vanadium et de phosphore, comportant les étapes suivantes:

(a) introduire un composé de vanadium pentavalent dans un milieu organique liquide de réaction choisi à partir d'alcools et de glycols capables de réduire au moins une portion du vanadium à un état de valence de +4;

(b) introduire au moins un composé de phosphore pentavalent dans le milieu de réaction;

(c) effectuer la réduction du vanadium en chauffant à reflux avant ou après l'addition du composé du phosphore et faire réagir le vanadium avec le composé du phosphore pour former un précurseur du catalyseur;

(d) récuper le précuseur du catalyseur en l'extrayant du milieu de réaction;

(e) sécher le précurseur du catalyseur; et

(f) calciner le précuseur du catalyseur; caractérisé en ce que (1) aucun agent réducteur corrosif n'est ajouté au milieu organique liquide de réaction de sorte que ce milieu n'est pas un solvant pour le précurseur du catalyseur, et (2) au cours de la réduction du vanadium par chauffage à reflux conformément à l'étape (c), on enlève au moins 1,5 mole du mileu organique liquide par mole de vanadium réduit ou ayant réagi.

2. Procédé selon la revendication 1, caractérisé en ce que l'on enlève de 1.5 à 15 moles de liquide organique par mole de vanadium réduit ou ayant réagi.

3. Procédé selon la revendication 1 ou la revendication 2, caractérisé en ce que le catalyseur comporte en outre des éléments promoteurs choisis parmi U, Co, Mo, Fe, Zn, Hf, Zr et leurs mélanges.

4. Procédé selon l'une quelconque des revendications 1 à 3, caractérisé en ce que l'on ajoute le composé du phosphore au milieu de réaction avant qu'une réduction substantielle du vanadium se produise.

5. Procédé selon l'une quelconque des revendications 1 à 4, caractérisé en ce que le composé du phosphore comprend de l'acide orthophosphorique.

6. Procédé selon l'une quelconque des revendications 1 à 5, caractérisé en ce que le composé du phosphore comprend un mélange d'acide orthophosphorique et d'acide pyrophosphorique.

7. Procédé selon l'une quelconque des revendications 1 à 6, caractérisé en ce que le liquide organique comprend de l'isobutanol et/ou de l'éthylène glycol.